(19) Europäisches Patentamt
European Patent Office
Office européen des brevets

(11) Veröffentlichungsnummer: **0 000 771**
**B1**

(12) # EUROPÄISCHE PATENTSCHRIFT

(45) Veröffentlichungstag der Patentschrift: **16.09.81**

(21) Anmeldenummer: **78100580.6**

(22) Anmeldetag: **02.08.78**

(51) Int. Cl.³: **C 07 C 31/02,**
C 07 C 29/00, A 61 K 7/46,
C 07 C 49/20,
C 07 C 49/04,
C 07 C 33/02, C 11 B 9/00

(54) **2,5,7-Trimethyloctan-3-ol und 2,4-Dimethylnonan-8-ol; Verfahren zu deren Herstellung, deren Verwendung als Riechstoffe; diese enthaltende Riechstoffkompositionen, sowie Herstellung solcher Riechstoffkompositionen.**

(30) Priorität: **04.08.77 LU 77923**
**15.06.78 CH 6537/78**

(43) Veröffentlichungstag der Anmeldung:
**21.02.79 Patentblatt 79/4**

(45) Bekanntmachung des Hinweises auf die Patenterteilung:
**16.09.81 Patentblatt 81/37**

(84) Benannte Vertragsstaaten:
**CH DE FR GB NL**

(56) Entgegenhaltungen:
**DE - B - 1 643 856**
**US - A - 3 833 635**

(73) Patentinhaber: **L. Givaudan & Cie Société Anonyme**
**Patentdienst Postfach**
**CH-4002 Basel (CH)**

(72) Erfinder: **Ochsner, Paul Albert, Dr.**
**30 Avenue des Tilleuls**
**CH-1203 Genf. (CH)**

(74) Vertreter: **Lederer, Franz, Dr. et al,**
**Patentanwälte Lederer, Meyer Lucile-Grahn-Strasse 22**
**D-8000 München 80 (DE)**

**EP 0 000 771 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 2,5,7-Trimethyloctan-3-ol und 2,4-Dimethylnonan-8-ol; Verfahren zu deren Herstellung, deren Verwendung als Riechstoffe; diese enthaltende Riechstoffkompositionen, sowie Herstellung solcher Riechstoffkompositionen

Die Erfindung betrifft neue Riechstoffe, nämlich Verbindungen der Formel

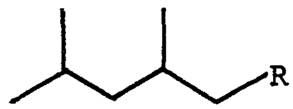

worin R den 1-Hydroxy-2-methyl-propyl- oder den 3-Hydroxy-butylrest darstellt.

Die Formel I umfasst demzufolge das 2,5,7-Trimethyloctan-3-ol (Ia) und das 2,4-Dimethyl-nonan-8-ol (Ib).

Die Erfindung betrifft auch ein Verfahren zur Herstellung dieser neuen Alkohole. Es ist dadurch gekennzeichnet, dass man entweder

a) 3,5-Dimethylhexanal mit einem Isopropylmagnesiumhalogenid bzw. Isobutyraldehyd mit einem 2,4-Dimethylpentylmagnesiumhalogenid umsetzt, oder dass man

b) 2,5,7-Trimethyl-4-octen-3-ol bzw. 6,8-Dimethyl-3-nonen-2-ol oder 2,5,7-Trimethyl-1,4-octadien-3-on bzw. 6,8-Dimethyl-3,5-nonadien-2-on oder 2,5,7-Trimethyl-3-octanon bzw. 6,8-Dimethyl-2-nonanon hydriert.

Die — ausschliesslich zu Ia führende — Umsetzung gemäss Verfahrensvariante a) kann nach den an sich bekannten Methoden der Grignardreaktion durchgeführt werden. Man arbeitet also zweckmässigerweise in Diäthyläther als Lösungsmittel und bei Temperaturen von 0—10°C.

Als Halogenid kommen Chlorid, Bromid und Jodid in Frage, besonders geeignet sind Bromid und Chlorid.

Die Hydrierungen gemäss Verfahrensvariante b) werden zweckmässigerweise katalytisch durchgeführt.

Im Falle des Octenols kommen als Katalysatoren zweckmässigerweise Raney-Nickel und Palladium in Frage.

Bevorzugt ist Palladium auf Kohle.

Man arbeitet zweckmässigerweise bei Raumtemperatur — doch kommen auch höhere oder niedrigere Temperaturen in Frage — und in Lösungsmitteln wie Aethylacetat, Aethanol, Aceton, etc.

Im Falle des Octanons bzw. des 1,4-Octadienons kommen insbesondere Kupferchromit oder Nickel als Katalysatoren in Frage.

Das Octanon kann schliesslich auch mittels Natriumborhydrid oder Lithiumaluminiumhydrid zum Alkohol reduziert werden (L.F. Fieser & M. Fieser, Reagents for Organic Synthesis, John Wiley & Sons 1967, 582, 1049).

Im Falle des 1,4-Octadienons arbeitet man zweckmässigerweise zunächst bei Raumtemperatur, um zunächst die Doppelbindungen zu hydrieren, und hierauf — zwecks Hydrierung der Ketogruppe — bei erhöhter Temperatur. Diese letztere Hydrierung kann z.B. bei Temperaturen von 120—150°C, insbesondere bei ca. 140°C erfolgen.

Im Falle des Nonenols geht man vorzugsweise von in situ hergestelltem Material aus, das beispielsweise durch katalytische Hydrierung aus 6,8-Dimethyl-3-nonen-2-on unter Verwendung von Kupferchromit zugäglich ist.

Auch das 3,5-Nonadienon wird vorzugsweise unter Verwendung von Kupferchromit hydriert.

Das Nonanon wird zweckmässigerweise unter Verwendung von Natriumborhydrid oder Lithiumaluminiumhydrid zum Alkohol Ib reduziert.

Die für die Verfahrensvariante a) benötigten 2,4-Dimethylpentylhalogenide sind durch Addition von Halogenwasserstoff an 2,4-Dimethyl-1-penten nach an sich bekannten Methoden — in Anwesenheit eines Peroxyds — oder durch Ersatz der primären alkoholischen Gruppierung von 2,4-Dimethylpentanol — ebenfalls in an sich bekannter Weise mittels Phosphorhalogeniden — zugänglich.

Die Herstellung von 2,5,7-Trimethyl-1,4-octadien-3-on ist beispielsweise aus 2,4,7-Trimethyl-7-octen-5-in-4-ol durch propargylische Umlagerung — in an sich bekannter Weise unter Zuhilfenahme eines Vanadium-Katalysators — möglich. Durch katalytische Hydrierung des erhaltenen Dienons bei Zimmertemperatur — wie vorne beschrieben — ist 2,5,7-Trimethyl-3-octanon zugänglich. Das 6,8-Dimethyl-3-nonen-2-on ist beispielsweise durch Aldolkondensation von 3,5-Dimethylhexanal mit Aceton zugänglich.

Die Alkohole I weisen besondere organoleptische Eigenschaften auf, auf Grund derer sie sich vorzüglich als Riechstoffe eignen.

Die Erfindung betrifft demgemäss auch die Verwendung von I, insbesondere in Form von Gemischen, als Riechstoffe, und Riechstoffkompositionen, die durch einen Gehalt an diesen Alkoholen I gekennzeichnet sind.

Die erfindungsgemäss als Riechstoff verwendete Verbindung Ia zeichnet sich durch interessante fruchtige (aprikosenartige) Geruchsnuancen aus. Der Alkohol Ib zeichnet sich andererseits durch eine angenehm blumige, fruchtige, erdig-grüne, pilzige Note aus, wobei aldehydrische Nebennoten festgestellt werden können. Die Verbindungen können demgemäss beispielsweise zur Parfümierung von Produkten, wie Kosmetika (Seifen, Mundwässern, Desodorantien, Shampoos, Lotions, Salben, Pudern, etc.), Detergentien, etc. dienen, wobei die Verbindungen I vorzugsweise nicht allein, sondern in Form von Kompositionen mit andern Riechstoffen eingesetzt werden.

Von grossem Vorteil ist die Tatsache, dass das 2,5,7-Trimethyl-octan-3-ol in Bouquets der verschiedensten Richtungen einen samtartigen, abrundenden Effekt bewirkt, der dabei noch wesentlich wichtiger sein kann als der aprikosenartige Eigengeruch. Mit dem 2,4,-Dimethyl-nonan-8-ol, das eine erdige, natürliche Note besitzt, erzielt man durch dessen Zusatz in Kompositionen eine erhebliche Verstärkung, und eine Strahlungskraft, die besonders in Rosen- und Jasminnoten zur Geltung kommt. Da es sich zudem bei den neuen Verbindungen um gesättigte Alkohole handelt, sind sie in Gemischen äusserst stabil.

Als Riechstoffe eignen sich die Verbindungen I auf Grund ihres hohen harmonischen Einfügungsvermögens, insbesondere in Kombination mit einer Reihe von natürlichen Riechstoffen, wie z.B. Galbanumöl, Vetiveröl, Patchouliöl, Zedernholzöl, Bergamotteöl, Fichtennadelöl, Petitgrainöl, Baummoos, Citronenöl, Corianderöl, Angelikasamenöl, Cardamomenöl.

Die Verbindung Ia kann auch vorteilhaft in Mischungen mit Mastixöl, Palmarosaöl, Jasmin Absolut, Mandarinenöl und Ysopöl verwendet werden, während die Verbindung Ib andererseits mit Ylang-Ylangöl erwünschte Effekte bewirkt.

Dank ihrer Strahlungskraft und Natürlichkeit können beide Verbindungen vorteilhaft für die Herstellung von Kompositionen mit z.B. folgenden synthetischen Riechstoffen verwendet werden:

— mit Aldehyden, wie Hydroxycitronellal, n-Decanal, 2,4-Dimethyl-3-cyclohexenyl-1-carboxaldehyd, substituierten Zimtaldehyden, 3,5,6-Trimethyl-3-cyclohexen-1-carboxaldehyd, p-tert.-Butyl-α-methylhydrozimtaldehyd, p-Isopropyl-α-methylhydrozimtaldehyd, p-Tolyl-acetaldehyd, α-Methyl-3,4-methylen-dioxy-hydrozimtaldehyd, 2,6-Dimethyl-5-heptenal, usw.
— mit Ketonen, wie 7-Acetyl-1,1,3,4,4,6-hexamethyltetrahydro-(1,2,3,4)-naphthalin, α-Jonon, Allyl-α-jonon, Ketonmoschus, p-Methylacetophenon, usw.
— mit Acetalen, wie 1-Phenyl-4-methyl-3,5-dioxaoctan, Phenyiacetaldehyddimethylacetal, usw.
— mit phenolischen Körpern, wie Eugenol, usw.
— mit Alkoholen, wie Phenyläthylalkohol, Linalool, Citronellol, cis-6-Nonenol, Zimtalkohol, Phenylpropyl-alkohol, Geraniol, Nerol, usw.
— mit Aethern, wie 1-Methylcyclododecan-1-yl-methyläther, 8α,12-Oxido-13,14,15,16-tetranorlabdan, usw.
— mit Estern, wie Benzylacetat, Aethyl-α-methylphenylglycidester, Linalylacetat, Vetivenylacetat, Linalylanthranilat, Methyldihydrojasmonat, Benzylsalicylat, 2-tert.-Butylcyclohexylacetat, Acetessigsäureäthylester, Dimethylbenzylmethylbutyrat, 5-[4- oder 5-(Acetoxymethyl)-1-cyclohexenyl]-2-methyl-2-penten, Hexenylsalicylat, Hexenylacetat, Benzylpropionat, Phenoxyäthylisobutyrat, p-Cresylphenylessigsäureester, Zimtformiat, Geranylacetat, Styrallylacetat, Phenyläthylformiat, usw.
— mit Lactonen, wie Cumarin, γ-Undecalacton, γ-Nonalacton, usw.
— mit schwefelhaltigen Verbindungen, wie p-Menthan-8-thiol-3-on, Sulfiden, usw.
— mit stickstoffhaltigen Verbindungen, wie Indol, 5-Methyl-3-heptanonoxim, Dimethylpyrazin, usw.

Die Verbindungen Ia und Ib können demgemäss bei der Herstellung von Kompositionen und — wie obige Herstellung zeigt — unter Verwendung einer breiten Palette bekannter Riechstoffe, insbesondere zur Herstellung von Kompositionen mit fruchtigen, blumigen, grünen, holzigen, chyperartigen und cologneartigen Noten verwendet werden.

Die unter Verwendung von I hergestellten Riechstoffkompositionen bestechen insbesondere durch die ausserordentliche Strahlungskraft, Natürlichkeit und Lebhaftigkeit.

So vermag z.B. Ia blumig-fruchtige Parfüm-Kompositionen durch zusätzliche Strahlungskraft und Natürlichkeit zu bereichern und kommt damit den Tendenzen der modernen Parfümerie in willkommener Weise entgegen. Der Alkohol Ia hebt in frischen, citrusartigen, holzigen Kompositionen die Hesperidennote in vorteilhafter Weise und unterstreicht zugleich den Holzcharakter auf veredelnde Art. Allgemein kann festgestellt werden, dass Ia beispielsweise sehr gute Effekte in ausgeprägt femininen Parfüms zeigt, aber andererseits auch vorteilhaft in maskulinen Kompositionen verwendet wird. Es können unter Zuhilfenahme von Ia auch interessante Blumenkomplexe hergestellt werden und Ia lässt sich gut mit Edelholznoten kombinieren.

Der Alkohol Ib weist einen natürlichen, pilzartigen, an feuchten Wald erinnernden Eigengeruch auf. Aufgrund dieser Eigenschaft erzielt man mittels Ib z.B. in Jasminoder Rosenkompositionen eine deutliche Verstärkung des erwünschten natürlichen Charakters des Parfums. Die Strahlungskraft, die die Verbindung Ib Kompositionen verleiht, ist besonders wertvoll bei der Herstellung von

Kompositionen, die in der industriellen Parfümerie verwendet werden, wie z.B. beim Parfümieren von Seifen und Waschpulvern.

Die Konzentration der Verbindungen I kann je nach dem Verwendungszweck innerhalb weiter Grenzen variieren, beispielsweise zwischen etwa 0.01 (Detergentien) und etwa 15 Gew.% (alkoholische Lösungen). In Parfümbasen bzw. Konzentraten können die Konzentrationen selbstverständlich auch höher liegen. Die Parfumbasen können in üblicher Weise zur Parfümierung von Eaux de Cologne, Eaux de toilette, Lotionen, Crèmes, Shampoos, Seifen und Detergentien, etc. verwendet werden.

Bei niedrigen Dosierungen (z.B. 0,5—2%) von I kann bereits eine deutliche Zunahme der Strahlungskraft festgestellt werden, ohne dass der Grundcharakter der Komposition wesentlich verändert wird. Bei höheren Dosierungen (z.B. 10—30%) tritt zusätzlich eine den olfaktischen Eigenschaften der verwendeten Verbindung entsprechende Modifizierung ein.

Beispiel 1

In einen Rundkolben, der mit Rührer, Kühler, Thermometer und Tropftrichter versehen ist, gibt man 26,4 g (1,1 Grammatom) Magnesiumspäne und 100 ml trockenen Aether. Man gibt hierauf langsam eine Lösung von 86,4 g (1,1 Mol) Isopropylchlorid in 150 ml wasserfreiem Aether zu und hält das Gemisch bei Rückflusstemperatur. Die Umsetzung wird durch Zugabe von wenig Jod zum Magnesium in Gang gebracht. Nach Beendigung der Zugabe des Isopropylchlorids hält man das Gemisch noch 1 Stunde bei Rückflusstemperatur. Man gibt nun langsam bei 5°C 128,2 g (1 Mol) 3,5-Dimethylhexanal, gelöst in 250 ml wasserfreiem Aether zu. Wiederum hält man das Gemisch — 3 Stunden — bei Rückflusstemperatur. Man kühlt hierauf auf 0°C ab und zersetzt das Reaktionsprodukt durch Zugabe von 340 ml eisgekühlter 10%iger Salzsäure. Die ätherische Lösung wird mittels Wasser neutral gewaschen, hierauf getrocknet und das Lösungsmittel abgedampft. Man erhält auf diese Weise 176 g (70%) Rohprodukt, dessen fraktionierte Destillation 121,4 g 2,5,7-Trimethyloctan-3-ol ergibt. Sdp. = 79°/5 mmHg, $n_D^{20} = 1,4362$.

Beispiel 2

a) In einen Rundkolben, der mit Rührer, Kühler, Thermometer und Tropftrichter versehen ist, gibt man 26,4 g (1,1 Grammatom) Magnesiumspäne und 100 ml wasserfreien Aether. Man gibt nun langsam eine Lösung von 90,2 g (1,15 Mol) Isopropylchlorid in 150 ml Aether zu. Nach Beendigung der Zugabe hält man das Reaktionsgemisch noch 1 Stunde bei Rückflusstemperatur. Man gibt hierauf bei einer Temperatur von 10°C 126,2 g (1 Mol) 3,5-Dimethyl-2-hexenal, gelöst in 250 ml wasserfreiem Aether, zu. Man hält 3 Stunden bei Rückflusstemperatur, kühlt hierauf auf 0°C ab und zersetzt durch Zugabe von 370 ml 10%iger, eisgekühlter Salzsäure. Die ätherische Lösung wird durch Zugabe von Wasser neutral gewaschen, hierauf getrocknet und das Lösungsmittel abgedampft. Man erhält auf diese Weise 168 g Rohmaterial, dessen fraktionierte Destillation 123,7 g (72,6%) 2,5,7-Trimethyl-4-octen-3-ol geben. Sdp. = 78—79°/5 mmHg, $n_D^{20} = 1,4526$.

b) 100 g des Octenols, gelöst in 200 ml Aethylacetat werden in Anwesenheit von 10 g 5%igem Palladium auf Kohle hydriert. Nach Aufnahme von 5,08 lt Wasserstoff bei 20°C (die theoretische Menge beträgt 14,6 lt) und bei Atmosphärendruck hört die Wasserstoffaufnahme auf. Man entfernt den Katalysator durch Filtration, verdampft das Lösungsmittel und beendigt die Hydrierung in einem Autoklaven in Anwesenheit von 1 g Raney-Nickel bei 100°C/10 Atm. Nach 2 Stunden lässt man Abkühlen, filtriert den Katalysator ab und destilliert das Produkt. Man erhält auf diese Weise 63 g 2,5,7-Trimethyl-octan-3-ol, Sdp. = 72°/5 mmHg, $n_D^{20} = 1,4362$, $d_4^{20} = 0,8287$. Die Ausbeute beträgt 62,5%.

Beispiel 3

In einen 2 1-Rundkolben, der mit Rührer, Kühler, Thermometer und Tropftrichter versehen ist, gibt man 400 ml wasserfreies Toluol und 200 g pulverförmiges Kaliumhydroxyd. Man kühlt auf —20°C und gibt nun bei einer Temperatur zwischen –20 und –10°C 66 g (1 Mol) 3-Methyl-3-buten-1-in [A.F. Thompson jun., N.A. Milas, Ida Rovno, J.Am.Chem.Soc. *63,* (1941), 752—755] zu. Man hält das Gemisch 1 1/2 Stunden unter Rühren bei einer Temperatur von —10°C und gibt hierauf langsam bei 0°C 110 g (1,1 Mol) Methyl-isobutylketon zu. Man rührt nun 1 Stunde bei 0°C und lässt hierauf innert 2 Stunden unter Weiterrühren die Temperatur auf 20°C ansteigen. Das Reaktionsgemisch wird auf Eis gegeben; man extrahiert mit 500 ml Toluol, wäscht mit Sole bis zur Neutralität und dampft das Lösungsmittel ab. Man erhält 183 g Rohprodukt, dessen Destillation 129,5 g (78,4%) 2,4,7-Trimethyl-7-octen-5-in-4-ol ergeben. Sdp. 76—77°/5 mmHg, $n_D^{20} = 1,4690$, $d_4^{20} = 0,8620$.

b) In einen 2 1-Rundkolben, der mit Thermometer, Rührer und Kühler versehen ist, gibt man 880 g Paraffinoel. Man erhitzt dieses bei einem Druck von 0,1 mmHg zwecks Entgasen 1 Stunde auf 140°C.

Nach Abkühlung auf 30°C gibt man 75 g Triphenylsilanol, 2 g Stearinsäure, 7,6 ml Triisopropylvanadat und 100 g des obigen Octeninols zu. Man erhitzt nun das Gemisch 2 Stunden auf

140°C, und zwar unter Stickstoffatmosphäre, und lässt wiederum auf 80°C abkühlen. Das gebildete rohe Keton destilliert man bei einer Temperatur von 50—70°C ab. Die Destillation wird weitergeführt, bis eine Innentemperatur von 150°C und ein Vakuum von 1 mmHg erreicht ist. Man erhält auf diese Weise 71 g (71%) des rohen 2,5,7-Trimethyl-1,4-octadien-3-on (z + e), $n_D^{20} = 1{,}4736$. Die Konstanten des destillierten reinen Produkts sind die folgenden: Sdp. = 77°/5 mmHg, $n_D^{20} = 1{,}4742$, $d_4^{20} = 0{,}8674$.

c) 16,6 g des rohen Ketons werden in 250 ml Methanol bei Atmosphärendruck und 20°C in Anwesenheit von 3 g Raney-Nickel und 1 g Natriumcarbonat hydriert. Nach Aufnahme von 4,84 1 Wasserstoff (theoretische Menge: 7,5 1) hört die Wasserstoffaufnahme auf. Man filtriert den Katalysator ab, dampft das Methanol ab und gibt das Reaktionsprodukt — enthaltend 2,5,7-Trimethyl-3-octanon — in einen Autoklaven, zusammen mit 3 g Raney-Nickel und 1 g Natriumcarbonat. Nach 48 Stunden bei 140°C und 40 Atmosphären/Wasserstoffdruck ist die Hydrierung beendigt. Man gewinnt 14 g (67%) Rohprodukt, dessen Destillation 11,5 g 2,5,7-Trimethyl-octan-3-ol liefern. Sdp. = 72°/5 mmHg, $n_D^{20} = 1{,}4362$, $d_4^{20} = 0{,}8287$.

## Beispiel 4

a) In einen rostfreien Stahlautoklaven (300 ml Fassungsvermögen) gibt man 112 g 2,4-Dimethyl-2-pentenal und 5 g Kupferchromit. Man hält das Gemisch bei einem Druck von 25 Atmosphären Wasserstoff und einer Temperatur von 150°C. Nach 8 Stunden ist die theoretische Wasserstoffmenge aufgenommen, nämlich 50 l (auf Atmosphärendruck berechnet). Man filtriert vom Katalysator ab und destilliert die erhaltenen 108 g Rohprodukt. Man erhält 90,7 g (78,3%) 2,4-Dimethylpentanol, Sdp. = 81°/34 mmHg, $n_D^{20} = 1{,}4232$, $d_4^{20} = 0{,}8199$.

b) In einen Rundkolben, der mit Rührer, Thermometer, Kühler und Tropftrichter versehen ist, gibt man 116 g (1 Mol) des obigen Pentanols und 12 g wasserfreies Pyridin. Man gibt nun langsam unter Kühlung 110 g (0,4 Mol) Phosphortribromid zu. Während der Zugabe hält man die Temperatur unterhalb 60°C, hält aber nach der Zugabe das Gemisch noch 1 Stunde bei 60°C. Nach Abkühlung nimmt man das Gemisch in Aether auf, wäscht die ätherische Lösung mit Wasser, hierauf mit einer 8%igen Natriumbicarbonatlösung und dann nochmals mit Wasser bis zur neutralen Reaktion. Man trocknet über wasserfreiem Natriumsulfat und dampft das Lösungsmittel ab. Man erhält 206 g rohes Bromid, dessen Destillation 111,5 g (62,3%) 2,4-Dimethylpentyl-bromid (1) ergeben. Sdp. = 70°/40 mmHg, $n_D^{20} = 1{,}4495$, $d_4^{20} = 1{,}1376$.

c) In einen Rundkolben, der mit Rührer, Thermometer, Kühler und Tropftrichter versehen ist, gibt man 9 g Magnesiumspäne und 50 ml wasserfreien Aether. Man gibt nun langsam beim Siedepunkt des Aethers eine Lösung von 72,2 g des obigen Bromids in 100 ml wasserfreiem Aether zu. Man erhitzt eine halbe Stunde auf Rückflusstemperatur und kühlt hierauf auf 10°C ab. Man fügt nun bei einer Temperatur von 10°C eine Lösung von 27,6 g Isobutyraldehyd in 60 ml getrocknetem Aether zu. Man bringt das Gemisch 3 Stunden auf Rückflusstemperatur und kühlt hierauf wiederum ab. Durch Zugabe von 20%iger Ammoniumchloridlösung wird das Reaktionsgemisch zersetzt. Man nimmt in Aether auf, wäscht sukzessive mit einer 10%igen Weinsäurelösung, mit Wasser, mit 10%iger Natriumcarbonatlösung, und hierauf nochmals mit Wasser bis zum Neutralpunkt. Man trocknet über wasserfreiem Natriumsulfat und dampft das Lösungsmittel ab. Man gewinnt 56,6 g Rohmaterial, dessen Destillation 32 g (46,2%) 2,5,7-Trimethyl-octan-3-ol ergeben. Sdp. = 78°C/6 mmHg. $n_D^{20} = 1{,}4370$, $d_4^{20} = 0{,}8287$.

## Beispiel 5

In einen Rundkolben, der mit Rührer, Kühler, Thermometer und Tropftrichter versehen ist, gibt man 128 g 3,5-Dimethylhexanal und 58 g Aceton. Das Gemisch wird auf 80°C erhitzt. Bei dieser Temperatur werden unter Rühren 150 ml 1n Natriumhydroxydlösung innerhalb einer Stunde zugetropft. Nach der Zugabe wird das Reaktionsgemisch noch 1/4 Stunde bei 80°C gehalten. Nach Erkalten wird in 200 ml Aether aufgenommen und mit Wasser bis zur Neutralität gewaschen. Nach dem Abdampfen des Lösungsmittels erhält man 147 g Rohprodukt, das durch Destillation fraktioniert wird. Die ersten Fraktionen (34 g) bestehen aus dem Ausgangsaldehyd (Sdp. 33°C/8 mmHg). Der zweite Teil (28 g) Des Destillats stellt das gewünschte Kondensationsprodukt 6,8-Dimethyl-3-nonen-2-on (Sdp. 88°C/6 mmHg) dar. Der dritte Teil (55 g) besteht aus dem Additionsprodukt 6,8-Dimethyl-4-hydroxynonan-2-on (Sdp. 106—108°C/6 mmHg), das wie folgt dehydratisiert wird:

55 g 6,8-Dimethyl-4-hydroxy-nonan-2-on werden in einem Claisenkolben über 2 g Kaliumhydrogensulfat unter einem Vakuum von 18 mmHg bei 92—103°C destilliert. Das Destillat wird in 100 ml Aether aufgenommen, mit Natriumhydrogencarbonatlösung, hierauf mit Wasser gewaschen. Nach dem Abdampfen des Lösungsmittels erhält man 43 g Rohprodukt, die durch Destillation 40 g 6,8-Dimethyl-3-nonen-2-on (Sdp. 88°C/6 mmHg) ergeben. Totalausbeute: 71 g 6,8-Dimethyl-3-nonen-2-on (42% der Theorie).

64 g des Ketons werden in einem Rührautoklaven in Gegenwart von 6 g Kupferchromit unter 40 bar Wasserstoffdruck hydriert. Die Temperatur wird zunächst auf 140°C gebracht. Wenn die Wasserstoffabsorption nachlässt (nach 3 Stunden), wird auf 170°C erhitzt und 3 Stunden zwischen 165 und 170°C gehalten. Nach dem Abkühlen auf 25°C wird vom Katalysator abgenutscht und destilliert. Man erhält 65 g chemisch reines 2,4-Dimethyl-nonan-8-ol (Ausbeute: 85,4%).

Beispiel 6

In einen 1 1-Stahlautoklaven, der mit einem Magnetrührer versehen ist (Autoclave Engineers, Inc.), gibt man 200 g frisch destilliertes 6,8-Dimethyl-3,5-nonadien-2-on und 2 g Kupferchromit. Man spült den Autoklaven mit Wasserstoff und setzt ihn zunächst unter einen Druck von 30 bar Wasserstoff. Man erhitzt nun rasch auf 100°C, hierauf langsam — innert 3 Stunden — auf 170°C. Der Druck wird alsdann auf 50 bar eingestellt, die Temperatur wird auf 200°C fixiert. Die theoretisch aufzunehmende Wasserstoffmenge beträgt 90 l. Man hält bei einem Druck von 50 bar und in einem Temperaturbereich zwischen 180—200°C. Nach 5 Stunden ist im Gemisch noch 7% ungesättigtes Material vorhanden. Man setzt die Hydrierung 6 weitere Stunden fort, um den Anteil an unhydriertem Produkt auf 0,2% herunterzudrücken. Das erhaltene Rohprodukt wird vom Katalysator abfiltriert und destilliert. Man erhält 171 g (Ausbeute 83%) chemisch reines 2,4-Dimethyl-nonan-8-ol vom Siedepunkt 91°/6 mmHg, $n_D^{20}$ = 1,4368; 136 g dieses Produktes werden als olfaktisch gut bezeichnet (Ausbeute 83% bzw. 65,7%).

Beispiel 7

| A. Base mit Fruchtcharakter | Gewichtsteile |
|---|---|
| Benzylacetat | 100 |
| $\alpha$-Amylzimtaldehyd | 100 |
| Phenyläthylalkohol | 100 |
| Fraise® (Aethyl $\alpha$-methylphenyl-glycidester) | 60 |
| Lilial® (p-tert.Butyl-$\alpha$-methylhydrozimtaldehyd) | 40 |
| Fixolid® (7-Acetyl-1,1,3,4,4,6-hexamethyltetrahydro (1,2,3,4)-naphthalin) | 80 |
| | 480 |

Das Gemisch wirkt vorerst "synthetisch", nicht abgerundet, wenn man dazu 100 Gewichtsteile 2,5,7-Trimethyloctan-3-ol gibt, erhält man eine sehr angenehme, reichhaltige, ausserordentlich natürliche Parfumkomposition, in der der fruchtige Charakter dominiert.

| B. Muguet-Base | Gewichtsteile |
|---|---|
| Cyclamenaldehyd® (p-Isopropyl-$\alpha$-methylhydrozimtaldehyd) | 10 |
| p-Tolylacetaldehyd | 5 |
| $\alpha$-Amylzimtaldehyd | 100 |
| Phenyläthylalkohol | 70 |
| Linalool | 50 |
| Linalylacetat | 50 |
| Fixolid® | 80 |
| Laurin® (Hydroxydihydro-citronellal) | 300 |
| | 665 |

Diese Komposition hat wohl Maiglöckchencharakter und zielt in Richtung Flieder; sie wirkt aber vorerst synthetisch und etwas agressiv. Nach Zugabe von 100 Gewichtsteilen 2,5,7-Trimethyl-octan-3-ol herrscht ein samtartiger Eindruck vor, die Note wirkt nun abgerundet und fruchtig.

| C. Fougère-Base | Gewichtsteile |
|---|---|
| Lavendelöl (franz.) | 60 |
| Linalylacetat | 50 |
| Cumarin | 50 |
| Phenyläthylalkohol | 80 |
| Hydroxycitronellal | 80 |
| Eichenmoos (jugoslawisch) | 30 |
| Vetivenylacetat® | 30 |
| Sandelholzöl | 40 |
| $\alpha$-Amylzimtaldehyd | 80 |
| Linalool | 60 |
| | 560 |

Wenn zu dieser Base 120 Gewichtsteile 2,5,7-Trimethyl-octan-3-ol gegeben werden, wird die grüne, agressive Note gedämpft, und es entsteht ein einheitlicher, abgerundeter, fruchtiger Geruch.

| D. Parfümerie-Base mit grüner Note | Gewichtsteile |
|---|---|
| Stemon (5-Methyl-3-heptanonoxim) | 5 |
| Galbanumöl | 5 |
| Corps Cassis® (8-Mercapto-p-menthan-3-on) (1 °/oo in Aethylcitrat) | 5 |
| Mastixöl absolut | 10 |
| Cyclal C® (2,4-Dimethyl-3-cyclohexenyl-1-carboxaldehyd) (10% in Propylenglykol) | 15 |
| Basilikumöl | 30 |
| Linalylanthranilat | 20 |
| Methyldihydrojasmonat | 50 |
| Benzylsalicylat | 90 |
| $\alpha$-Hexylzimtaldehyd | 90 |
| Linalylacetat | 90 |
| CD-Acetal® (Phenylacetaldehyd-glycerinacetal) | 40 |
| 2,5,7-Trimethyloctan-3-ol | 50 |
| | 500 |

Die Anwesenheit von 2,5,7-Trimethyloctan-3-ol erteilt der Komposition einen angenehmen, abgerundeten Charakter, der ohne diesen Alkohol eindeutig fehlt.

| E. Chypre-Base | Gewichtsteile |
|---|---|
| Corianderöl | 20 |
| Madrox® (1-Methylcyclododecan-1-yl-methyläther) | 65 |
| Patchouliöl | 30 |
| Hydroxycitronellal | 30 |
| Bergamotteöl | 50 |
| Fichtennadelöl | 20 |
| Galbanumöl | 20 |
| Angelikasamenöl | 10 |
| Cardamomenöl | 5 |
| $\alpha$-Jonon | 25 |
| Citronenöl (italien.) | 15 |
| Baummoos (50% in Propylenglykol) | 30 |
| Petitgrainöl | 20 |
| Helional® ($\alpha$-Methyl-3,4-methylendioxy-hydrozimtaldehyd) | 20 |
| Vetiveröl Bourbon | 5 |
| Citronellol | 10 |
| Zedernholzöl | 20 |
| Cumarin | 5 |
| 2,5,7-Trimethyloctan-3-ol | 100 |
| | 500 |

Zugabe von 2,5,7-Trimethyl-3-octanol verstärkt die fruchtige Note der Komposition. Sie wirkt voller, kräftiger und erweckt einen abgerundeten, angenehmen Eindruck.

| F. Parfümeriebase mit Aprikosennote | Gewichtsteile |
|---|---|
| $\alpha$-Jonon | 80 |
| Dimethylbenzylmethylbutyrat | 50 |
| Allyl-$\alpha$-jonon | 40 |
| Acetessigsäureäthylester | 30 |
| Palmarosaöl | 20 |
| $\gamma$-Undecalacton | 15 |
| Galaxolid® (1,3,4,6,7,8-Hexahydro-4,6,6,7,8,8-hexamethyl-cyclopenta-$\gamma$-2-benzopyran) | 10 |
| Propylenglykol | 230 |
| 2,5,7-Trimethyloctan-3-ol | 25 |
| | 500 |

Auch ohne 2,5,7-Trimethyloctan-3-ol weist diese Komposition einen fruchtigen Charakter auf; der Zusatz an 2,5,7-Trimethyloctan-3-ol verstärkt und verbessert aber die fruchtige Note in Richtung Aprikosen, der natürliche Effekt von reifen Aprikosen wird erzielt.

| G. Parfümeriebase mit Melonennote | Gewichtsteile |
|---|---|
| Myraldylacetat (5-[4- oder 5-(Acetoxymethyl)-1-cyclohexenyl]-2-methyl-2-penten) | 80 |
| Hexenylsalicylat | 70 |
| Cyclamenaldehyd® | 60 |
| Acetessigsäureäthylester | 50 |
| cis-6-Nonenol (10% in Propylenglykol) | 30 |
| Hexenylacetat (10% in Propylenglykol) | 20 |
| Melonal® [2,6-Dimethyl-5-heptenal] (10% in Propylenglykol) | 50 |
| Lilial® | 20 |
| Propylenglykol | 20 |
| 2,5,7-Trimethyloctan-3-ol | 100 |
| | 500 |

Die 100 Gewichtsteile 2,5,7-Trimethyloctan-3-ol vermitteln dem Gemisch einen natürlichen, abgerundeten Charakter, die typische Note der reifen Melone ist besser erkennbar.

| H. Agrumenartige Base | Gewichtsteile |
|---|---|
| Bergamotteöl | 300 |
| Corps Cassis® [8-Mercapto-p-menthan-3-on (1 °/oo in Aethylcitrat) | 300 |
| Mandarinenöl | 150 |
| Galbanumöl | 120 |
| Dimethylpyrazin (10% in Propylenglykol) | 10 |
| 2,5,7-Trimethyloctan-3-ol | 120 |
| | 1000 |

Ohne Zusatz von 2,5,7-Trimethyloctan-3-ol wirkt die Mischung "spitzig" und agressiv; mit dem neuen Alkohol erhält man eine abgerundete, viel angenehmer wirkende Komposition, die sich für die Parfümerie besonders gut eignet.

| I. Salbeiöl ähnliche Base | Gewichtsteile |
|---|---|
| Bergamotteöl | 120 |
| Ysopöl | 80 |
| Patchouli Anhydrol | 80 |
| Allyl-$\alpha$-jonon | 30 |
| $8\alpha$,12-Oxido-13,14,15,16-tetranorlabdan | 5 |
| 2,5,7-Trimethyloctan-3-ol | 185 |
| | 500 |

Mit 185 Teilen 2,5,7-Trimethyloctan-3-ol erhält man eine Mischung mit einem originellen, den Salbeicharakter eindeutig betonenden Effekt, die Note ist in der modernen Parfümerie gesucht.

| K. Jasmin-Base | Gewichtsteile |
|---|---|
| Benzylacetat | 400 |
| α-Hexylzimtaldehyd | 200 |
| Benzylpropionat | 50 |
| Ylang-Ylang-Oel | 50 |
| Linalool | 50 |
| Phenoxyäthylisobutyrat | 50 |
| Phenyläthylalkohol | 30 |
| p-Cresyl-phenylessigsäureester (10% in Propylenglykol) | 30 |
| Undecalacton (10% in Propylenglykol) | 20 |
| Indol (10% in Propylenglykol) | 20 |
| | 900 |

Wenn zu dieser klassischen Jasminbase 10C Gewichtsteile 2,4-Dimethyl-nonan-8-ol gegeben werden, entsteht eine Base, die nun nicht nur viel natürlicher wirkt, sondern auch einen abgerundeteren blumigen Charakter aufweist.

Diese blumige Base eignet sich besonders gut für die Parfümierung von Seifen und Waschpulvern. Bei einer Dosierung von 1 bis 1,5% Gewichtsteilen in Seifen verhält sich die Komposition wie ein äusserst stabiles kräftiges Parfum. In Waschpulvern wird die optimale Wirkung bei einer Dosierung von 0,1 bis 0,3% erhalten; die blumige Note wird so durch die Anwesenheit von 2,4-Dimethyl-nonan-8-ol besonders hervorgehoben.

| L. Komposition "Rose" | Gewichtsteile |
|---|---|
| Phenyläthylalkohol | 300 |
| Geraniol | 200 |
| Citronellol | 200 |
| Fixolid® (7-Acetyl-1,1,3,4,4,6-hexamethyltetrahydro (1,2,3,4)-naphthalin) | 100 |
| Nerol | 50 |
| Phenylessigsäure | 5 |
| | 855 |

Diese Komposition stellt eine "gewöhnliche" Rosenkomposition dar. Wenn man nun 145 Gewichtsteile 2,4-Dimethyl-nonan-8-ol zugibt, erhält man eine Base mit viel mehr Volumen, die natürlicher wirkt und besonders an Rosenblütenblätter und an die "Rose de mai" (Kreuzung zwischen Rosa centifolia und Rosa gallica) erinnert.

| M. Chypre-Base | Gewichtsteile |
|---|---|
| Corianderöl | 40 |
| Madrox® (1-Methylcyclododecan-1-yl-methyläther) | 130 |
| Patchouliöl | 60 |
| Hydroxycitronellal | 60 |
| Bergamotteöl | 100 |
| Fichtennadelöl | 40 |
| Galbanumöl | 40 |
| Angelikasamenöl | 20 |
| Cardamomenöl | 10 |
| $\alpha$-Jonon | 50 |
| Citronenöl (italien.) | 30 |
| Baummoos (50% in Propylenglykol) | 60 |
| Petitgrainöl | 40 |
| Helional® ($\alpha$-Methyl-3,4-methylendioxy-hydrozimtaldehyd) | 40 |
| Vetiveröl Bourbon | 10 |
| Citranellol | 20 |
| Zedernholzöl | 40 |
| Cumarin | 10 |
| | 800 |

Diese klassische Chypre-Base wird durch Zugabe von 100 Gewichtsteilen 2,4-Dimethyl-nonan-8-ol eindeutig verbessert, indem so eine blumige und grüne Nuance entsteht, und der (erwünschte) Geruch von Unterholz verstärkt wird.

| N. Fliederbase | Gewichtsteile |
|---|---|
| Terpineol | 260 |
| Hydroxycitronellal | 200 |
| Phenyläthylalkohol | 160 |
| Zimtalkohol (Substitut) | 100 |
| Phenylpropylalkohol | 100 |
| Zimtformiat | 20 |
| Geranylacetat | 10 |
| Ketonmoschus | 10 |
| Jasminölersatz | 10 |
| Eugenol | 5 |
| Indol (10% in Propylenglykol) | 5 |
| p-Methylacetophenon | 5 |
| γ-Undecalacton | 5 |
| Corps Cassis® [8-Mercapto-p-menthan-3-on] (1 °/oo in Aethylcitrat) | 5 |
| n-Decanal (10% in Propylenglykol) | 5 |
| 2,4-Dimethyl-nonan-8-ol | 100 |
| | 1000 |

Durch Zugabe von 100 Gewichtsteilen des neuen Alkohols erhält die konventionelle Flieder-Komposition eine erdige Nuance, welche den sonst "synthetisch" wirkenden Geruch ausgewogener, blumiger, natürlicher macht.

| O. Kölnisch Wasser für Herren | Gewichtsteile |
|---|---|
| Linalool | 50 |
| Angelikasamenöl | 10 |
| Cardamomenöl | 5 |
| Fichtennadelöl | 50 |
| Hydroxycitronellal | 70 |
| α-Jonon | 30 |
| Bergamotteöl | 80 |
| Patchouliöl | 30 |
| Baummoos (50% in Propylenglykol) | 30 |
| Cyclal C® [2,4-Dimethyl-3-cyclohexenyl-1-carboxaldehyd] (10% in Propylenglykol) | 5 |
| Styrallyiacetat | 5 |
| Petitgrainöl | 20 |
| Citronellol | 25 |
| Madrox® (1-Methylcyclododecan-1-yl-methyläther) | 50 |
| 2,4-Dimethyl-nonan-8-ol | 40 |
| | 500 |

In dieser Komposition bewirkt der neue Alkohol einen abrundenden Effekt. Die Komposition mit dem neuen Alkohol wirkt weniger "scharf", aber kräftiger, sie ist für die Parfümerie weit besser geeignet.

| P. Parfümeriebase mit grüner Note | Gewichtsteile |
|---|---|
| α-Hexylzimtaldehyd (Substitut) | 200 |
| Phenyläthylformiat | 200 |
| Galbanumöl (synthetisch) | 60 |
| Acetal R® (1-Phenyl-4-methyl-3,5-dioxaoctan) | 60 |
| Isocyclocitral® (3,5,6-Trimethyl-3-cyclohexen-1-carboxaldehyd) | 60 |
| 2-tert.-Butylcyclohexylacetat | 40 |
| Cyclal C® [2,4-Dimethyl-3-cyclohexenyl-1-carboxaldehyd] (10% in Propylenglykol) | 20 |
| Stemon® (5-Methyl-3-heptanonoxim) | 10 |
| Citronenöl | 10 |
| Propylenglykol | 140 |
| 2,4-Dimethyl-nonan-8-ol | 200 |
| | 1000 |

Abwesenheit von 2,4-Dimethyl-8-nonanol in der Komposition erweckt wohl einen blumigen, aber "synthetischen", "chemischen" Eindruck. Der Zusatz von 2,5-Dimethyl-nonan-8-ol verleiht der Base einen erdigen, natürlichen Charakter.

**Patentansprüche**

1. Verfahren zur Herstellung von Verbindungen der Formel

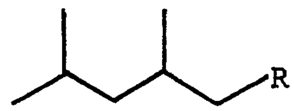

worin R den 1-Hydroxy-2-methyl-propyl- oder den 3-Hydroxy-butylrest darstellt, dadurch gekennzeichnet, dass man entweder

a) 3,5-Dimethylhexanal mit einem Isopropylmagnesium-halogenid bzw. Isobutyraldehyd mit einem 2,4-Dimethylpentylmagnesiumhalogenid umsetzt, oder dass man

b) 2,5,7-Trimethyl-4-octen-3-ol bzw. 6,8-Dimethyl-3-nonen-2-ol oder 2,5,7-Trimethyl-1,4-octadien-3-on bzw. 6,8-Dimethyl-3,5-nonadien-2-on oder 2,5,7-Trimethyl-3-octanon bzw. 6,8-Dimethyl-2-nonanon hydriert.

2. Riechstoffkomposition gekennzeichnet durch einen Gehalt an einer Verbindung der Formel

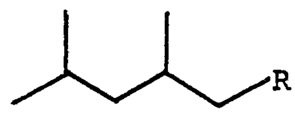

worin R den 1-Hydroxy-2-methyl-propyl- oder den 3-Hydroxy-butylrest darstellt.

3. Verbindungen der Formel

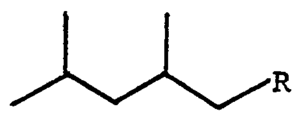

worin R den 1-Hydroxy-2-methyl-propyl- oder den 3-Hydroxy-butylrest darstellt.

**Revendications**

1. Procédé de préparation de composés de formule

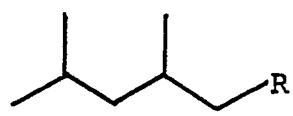

où R représente le radical 1-hydroxy-2-méthyl-propyle ou le radical 3-hydroxy-butyle, caractérisé en ce que, ou bien

a) On fait réagir du 3,5-diméthylhexanal avec un halogénure d'isopropylmagnésium ou de l'isobutyraldéhyde avec un halogénure de 2,4-diméthylpentylmagnésium, ou

b) On hydrogène du 2,5,7-triméthyl-4-octén-3-ol, ou du 6,8-diméthyl-3-nonén-2-ol ou de la 2,5,7-triméthyl-1,4-octadién-3-one ou de la 6,8-diméthyl-3,5-nonadién-2-one ou encore de la 2,5,7-triméthyl-3-octanone ou de la 6,8-diméthyl-2-nonanone.

2. Composition odoriférante, caractérisée en ce qu'elle contient un composé de formule

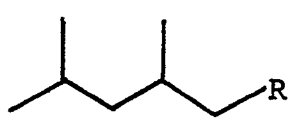

où R représente le radical 1-hydroxy-2-méthylpropyle ou le radical-3-hydroxy-butyl.

3. Composés de formule

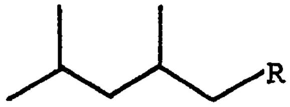

où R représente le radical 1-hydroxy-2-méthylpropyle ou le radical 3-hydroxybutyle.

**Claims**

1. Process for the manufacture of compounds of the formula

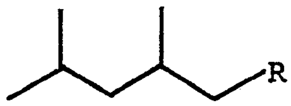

wherein R represents the 1-hydroxy-2-methyl-propyl residue of the 3-hydroxy-butyl residue, characterised by either

a) reacting 3,5-dimethylhexanal with an isopropylmagnesium halide or reacting isobutyraldehyde with a 2,4-dimethylpentylmagnesium halide, or

b) hydrogenating 2,5,7-trimethyl-4-octen-3-ol or 6,8-dimethyl-3-nonen-2-ol, or 2,5,7-trimethyl-1,4-octadien-3-one or 6,8-dimethyl-3,5-nonadien-2-one, or 2,5,7-trimethyl-3-octanone or 6,8-dimethyl-2-nonanone.

2. Odorant substance composition, characterised in that it contains a compound of the formula

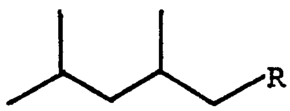

wherein R represents the 1-hydroxy-2-methyl-propyl residue or the 3-hydroxy-butyl residue.

3. Compounds of the formula

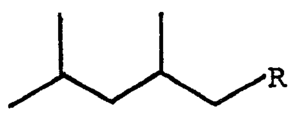

wherein R represents the 1-hydroxy-2-methyl-propyl residue or the 3-hydroxy-butyl residue.